# EUROPÄISCHE PATENTSCHRIFT

(11) **EP 1 208 798 B1**
(45) Veröffentlichungstag und Bekanntmachung des Hinweises auf die Patenterteilung: **06.04.2005**
(21) Anmeldenummer: 01123255.0
(22) Anmeldetag: 02.10.2001
(51) Int. Cl.: A61B 6/04

(54) **Röntgendiagnostikgerät**
Diagnostic x-ray apparatus
Appareil de diagnostic à rayons x

(30) Priorität: 23.11.2000 SE 0004298
(43) Veröffentlichungstag der Anmeldung: 29.05.2002
(73) Patentinhaber: Siemens Aktiengesellschaft, 80333 München (DE)
(72) Erfinder: Lindström, Krister, 125 34 Älvsjö (SE); Sundkvist, Helena, 168 57 Bromma (SE); Modig, Eva, 169 72 Solna (SE)

(56) Entgegenhaltungen:
- DE-A- 19 834 585
- US-A- 3 971 950
- US-A- 5 029 193
- US-A- 5 506 877
- US-A- 5 706 327

## Beschreibung

Die Erfindung bezieht sich auf ein Röntgendiagnostikgerät für Mammographieuntersuchungen mit einem Arm für eine Röntgenröhre und einem Objekttisch und einer zwischen der Röntgenröhre und dem Objekttisch angeordneten Kompressionsplatte, die über eine Halterung mit dem Arm verbunden und entlang diesem verschiebbar ist, wobei die Kompressionsplatte mit der Halterung derart verbunden ist, daß die Kompressionsplatte gegenüber dem Objekttisch in Längs- und Querrichtung der Platte drehbar ist.

Um bei einer Mammographieuntersuchung die bestmögliche Brustbildauflösung mit einer optimal niedrigen Röntgenstrahlendosis bei einer Aufnahme zu erhalten, ist es notwendig, daß die Kompressionsplatte gegen die zu untersuchende Brust, die am Objekttisch anliegt, mit einer Kraft gedrückt wird, die über die gesamte Brust gleich verteilt ist.

Da die Brust am häufigsten an dem brustkorbnahen Ende dicker ist als an dem Brustwarzenende, hat man gemäß der US-PS 5 706 327 versucht, das Problem mit der Gleichverteilung der Kompressionskraft zu lösen, indem die Kompressionsplatte mit der Halterung derart drehbar verbunden ist, daß die Kompressionsplatte gegenüber dem Objekttisch in dessen Längsrichtung drehbar ist. Dies erfolgt, indem die Halterung der Kompressionsplatte entlang einem Teil der beiden Längsseiten der Kompressionsplatte verläuft und daß die Kompressionsplatte mit der Halterung über Wellen verbunden ist, die an jeweils einer Längsseite angeordnet sind. Wenn die Kompressionsplatte gegen die Brust, die untersucht werden soll, gedrückt wird, wird die Kompressionsplatte um die erwähnten Wellen gedreht und drückt daher sowohl gegen das brustkorbnahe Ende als auch gegen das Brustwarzenende. Ein Nachteil dieses Aufbaus besteht darin, daß die Teile der Brust, die seitlich herausgedrückt werden, nicht notwendigerweise mit derselben Kompressionskraft zusammengepresst werden wie die Teile der Brust, die durch die Kompressionsplatte zuerst zusammengedrückt werden.

Ein Röntgendiagnostikgerät der eingangs genannten Art ist in der US-PS 5 506 877 dargestellt und beschrieben. Die Halterung der Kompressionsplatte ist U-förmig ausgebildet und erstreckt sich entlang der Querseite der Kompressionsplatte sowie entlang deren beider Längsseiten. Die Kompressionsplatte ist drehbar mit der Halterung über Wellen verbunden, die an den freien Enden der Halterung angebracht sind. Wenn die Kompressionsplatte und die Halterung mittels bekannter Mittel gegen die Brust, die untersucht werden soll, verschoben wird, ist die Kompressionsplatte parallel zu dem Objekttisch angeordnet. Wenn die Kompressionsplatte die Brust erreicht, wird sie um die erwähnten Wellen in ihre Längsrichtung mittels in der US-Schrift nicht beschriebener Mittel derart gedreht, daß die Kompressionsplatte sowohl gegen das brustkorbnahe Ende als auch gegen das Brustwarzenende der Brust drückt. Die Halterung und damit auch die Kompressionsplatte ist auch mittels eines Motors in Querrichtung um die Zentrumachse der Kompressionsplatte drehbar. Durch diese Möglichkeit, die Kompressionsplatte auch senkrecht zur Zentrumachse drehen zu können, kann, wenn die Brust am Objekttisch richtig plaziert wird, ein gleich verteilter Druck über die gesamte Brust erhalten werden. Die Brust richtig zu plazieren bedeutet, daß die fiktive Zentrumachse der Brust entlang der Zentrumachse der Kompressionsplatte, um die die Platte drehbar ist, verlaufen soll. Der Nachteil des hier beschriebenen Röntgendiagnostikgerätes ist es, daß sämtliche Bewegungen der Kompressionsplatte mit Motoren angetrieben werden, was das Gerät in der Herstellung teuer macht.

Der Erfindung liegt die Aufgabe zugrunde, ein Röntgendiagnostikgerät für Mammographieuntersuchungen der eingangs genannten Art mit einer Kompressionsplatte zu schaffen, die mit Hilfe von einfachen und damit verhältnismäßig billigen Mitteln einen Druck gegen die Brust, die untersucht werden soll, erzeugt, der auf optimale Weise automatisch gleich über die Brust verteilt wird.

Die Aufgabe wird erfindungsgemäß durch die Merkmale des Anspruchs 1 gelöst. Die Kompressionsplatte ist mittels mindestens eines federnden Verbindungsmittels in mindestens einem Verbindungspunkt mit der Halterung verbunden. Mit Hilfe des federnden Verbindungsmittels kann die Kompressionsplatte bei einer Kompression einer Brust automatisch , ohne Hilfe von Motoren in den erwähnten Längs- und Querrichtungen derart gedreht werden, daß eine optimale Verteilung der Kraft gegen die Brust erreicht wird. Hierdurch ist bei einer Aufnahme eine hohe Bildauflösung mit einer verhältnismäßig niedrigen Röntgenstrahlendosis gegeben.

In einer extrem einfachen Ausführungsform der Kompressionsplatte nach der Erfindung wird vorgeschlagen, daß ein einziges Verbindungsmittel in der Mitte derjenigen Seite der Kompressionsplatte, die gegen die Halterung gerichtet ist, angeordnet ist. Hierdurch werden die bereits oben genannten Vorteile erreicht, insbesondere dann, wenn die zu untersuchende Brust so placiert ist, daß die fiktive Zentrumachse der Brust entlang oder in der Nähe der Zentrumachse der Kompressionsplatte, um die Platte drehbar ist, verläuft.

In einer vorteilhaften Ausführungsform der Kompressionsplatte nach der Erfindung wird vorgeschlagen, daß zwei Verbindungsmittel auf jeweils einer Seite der Mitte der gegen die Halterung gerichteten Seite der Kompressionsplatte angeordnet sind. Hierdurch ist außer den bereits erwähnten Vorteilen ein weiterer Vorteil gegeben, indem die Brust nicht in eine besondere Position gegenüber der Kompressionsplatte gebracht werden muß. Dadurch, daß die Kompressionsplatte mit der Halterung in zwei mit Abstand von einander angeordneten federnden Verbindungspunkten verbunden ist, weist die Kompressionsplatte keine definierte Drehachse auf.

Nach der Erfindung wird weiterhin vorgeschlagen, daß die Halterung U-förmig ausgebildet ist und entlang der einen Querseite der Kompressionsplatte sowie entlang deren beiden Längsseiten verläuft, wobei jede Längsseite der Kompressionsplatte mit der Halterung mittels mindestens eines Verbindungsmittels verbunden ist. Hiermit ist gemeint, daß die Kompressionsplatte vorzugsweise mit der Halterung in vier Punkten verbunden werden kann. Die weiteren zwei Verbindungsmittel können vorzugsweise mit Abstand von einander an der Querseite der Kompressionsplatte angebracht sein.

In einer weiteren, sehr vorteilhaften Ausführungsform der Kompressionsplatte nach der Erfindung wird vorgeschlagen, daß die Halterung U-förmig ausgebildet ist und entlang der einen Querseite der Kompressionsplatte sowie entlang deren beiden Längsseiten verläuft, wobei jede Längsseite der Kompressionsplatte mit der Halterung mittels zweier Verbindungsmittel verbunden ist. Die Verbindungsmittel sind vorzugsweise in der Nähe der Ecken der Kompressionsplatte angeordnet. Die Anzahl der federnden Verbindungsmittel und deren Plazierungen führt dazu, daß diese Ausführungsform der Kompressionsplatte mit Rück-sicht auf eine automatische Verteilung des Druckes über die Brust bei einer Kompression optimal ist, unabhängig davon, wo die Brust am Objekttisch plaziert worden ist, da die Kompressionsplatte eine flexible, nicht definierte Drehachse aufweist.

In einer vorteilhaften Ausführungsform der Erfindung wird vorgeschlagen, daß die Halterung mit mindestens einer Gummibuchse versehen ist und daß die Kompressionsplatte mit der Gummibuchse über eine Welle, die an der Kompressionsplatte befestigt ist, verbunden ist.

In einer weiteren Ausführungsform der Erfindung wird vorgeschlagen, daß die Kompressionsplatte mit mindestens einer Gummibuchse versehen ist und daß die Halterung mit der Gummibuchse über eine Welle, die an der Halterung befestigt ist, verbunden ist. In Verbindung mit einer Kompression wird jede Welle bei einer Drehung der Kompressionsplatte in deren Längs- und Querrichtung in jeder Gummibuchse in der Höhe verschoben, wobei eine Deformierung der jeweiligen Gummibuchse erfolgt. Nach einer beendeten Kompression geht jede Gummibuchse automatisch in ihre ursprüngliche Form zurück.

Die Erfindung wird in Verbindung mit mehreren in den Figuren dargestellten Ausführungsbeispielen näher erläutert. Es zeigen:
- FIG 1: eine Seitenansicht eines Röntgendiagnostikgerätes für Mammographieuntersuchungen mit einer Halterung und einer Kompressionsplatte nach der Erfindung,
- FIG 2: eine Seitenansicht einer Halterung und eine Kompressionsplatte nach der FIG 1 in zwei verschiedenen Lagen,
- FIG 3: eine Vorderansicht einer Halterung und einer Kompressionsplatte nach der FIG 1 und 2,
- FIG 4: ein federndes Verbindungsmittel nach der Erfindung in einem Schnitt entlang der Schnittlinie IV-IV in der FIG 2,
- FIG 5: ein federndes Verbindungsmittel entlang der Schnittlinie V-V in der FIG 4,
- FIG 6: eine Draufsicht einer Halterung und einer Kompressionsplatte nach den FIG 1 bis 3 mit schematisch dargestellten Verbindungsmitteln,
- FIG 7: eine Draufsicht einer zweiten Halterung und einer Kompressionsplatte nach der Erfindung mit schematisch dargestellten Verbindungsmitteln,
- FIG 8: eine Draufsicht einer dritten Halterung und einer Kompressionsplatte nach der Erfindung mit schematisch dargestellten Verbindungsmitteln und
- FIG 9: eine Draufsicht einer vierten Halterung und einer Kompressionsplatte nach der Erfindung mit einem schematisch dargestellten Verbindungsmittel.

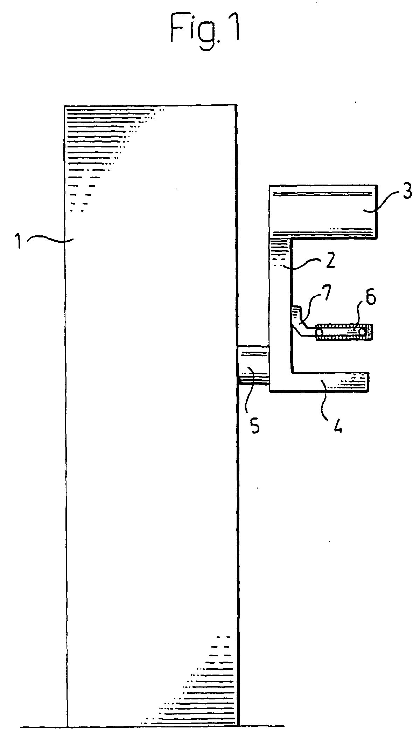

In der FIG 1 ist ein Röntgendiagnostikgerät für Mammographieuntersuchungen schematisch, mit einem Stativ 1, das einen Arm 2 für eine Röntgenröhre 3 und einen Objekttisch 4 trägt, gezeigt. Der Arm 2 ist über eine horizontal angeordnete Welle 5 mit dem Stativ 1 drehbar verbunden. Zwischen der Röntgenröhre 3 und dem Objekttisch 4 ist eine Kompressionsplatte 6 angeordnet, die über eine Halterung 7 mit dem Arm 2 verbunden und entlang diesem verschiebbar ist.

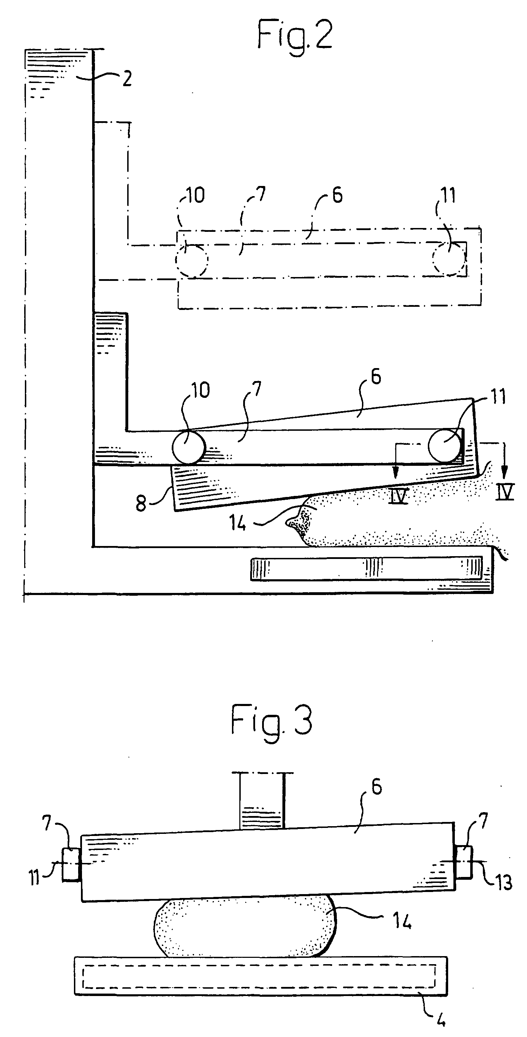

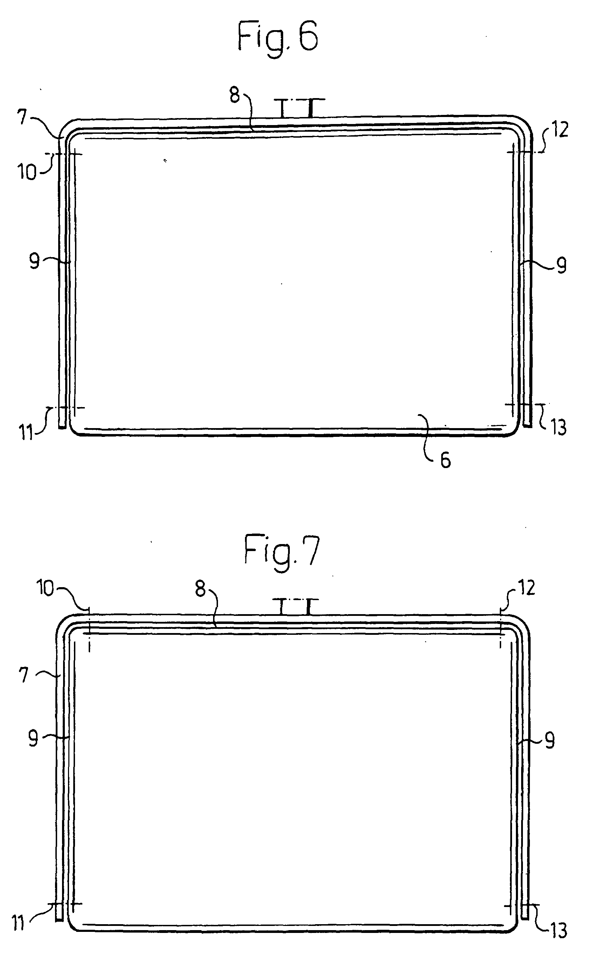

In der FIG 2 ist die Halterung 7 mit der Kompressionsplatte 6 gezeigt, bei der die Halterung 7 U-förmig ausgebildet ist und entlang der einen Querseite 8 der Kompressionsplatte 6 sowie entlang deren beiden Längsseiten 9 verläuft, wobei jede Längsseite 9 der Kompressionsplatte 6 mit der Halterung 7 mittels zwei federnden Verbindungsmitteln 10 bis 13 verbunden ist. In der FIG 6, die eine Draufsicht der Halterung 7 und der Kompressionsplatte 6 ist, ist eine optimale Plazierung der Verbindungsmittel 10 bis 13 gezeigt, indem sie an den Enden jeder Längsseite 9 angeordnet sind. In dieser FIG 6 sind die Verbindungsmittel 10 bis 13 lediglich schematisch dargestellt. In der FIG 2 ist die Halterung 7 und die Kompressionsplatte 6 in einer Parklage gezeigt. Die strichpunktierten Konturen der Halterung 7 und der Platte 6 zeigen diese Lage. In Verbindung mit einer Untersuchung einer Brust 14, die am Objekttisch 3 angebrächt ist, wird die Halterung 7 und dadurch auch die Kompressionsplatte 6 entlang dieses Armes 2 verschoben. Wenn die Kompressionsplatte 6 das brustkorbnahe Ende der Brust 14 erreicht hat, wird dieses Ende komprimiert, wobei sich die Kompressionsplatte 6 aufgrund der federnden Verbindungsmittel 10 bis 13 in ihrer Längsrichtung so dreht, daß die Platte 6 die gesamte Brust 14 bis zum Brustwarzenende komprimiert. Die Kompressionslage der Platte 6 ist in der FIG 2 gezeigt.

Durch die federnden Verbindungsmittel 10 bis 13 kann die Kompressionsplatte 6, wie in der FIG 3 gezeigt ist, auch im Verhältnis zur Halterung 7 in ihrer Querrichtung gedreht werden. Durch die hier beschriebene Aufhängung der Kompressionsplatte 6 wird ein gleich verteilter Kompressionsdruck über die gesamte Brust 14, unabhängig von der Plazierung der Brust 14 am Objekttisch 3, erhalten, was bei einer Aufnahme eine extrem gute Bildauflösung mit einer optimal niedrigen Röntgenstrahlendosis ergibt.

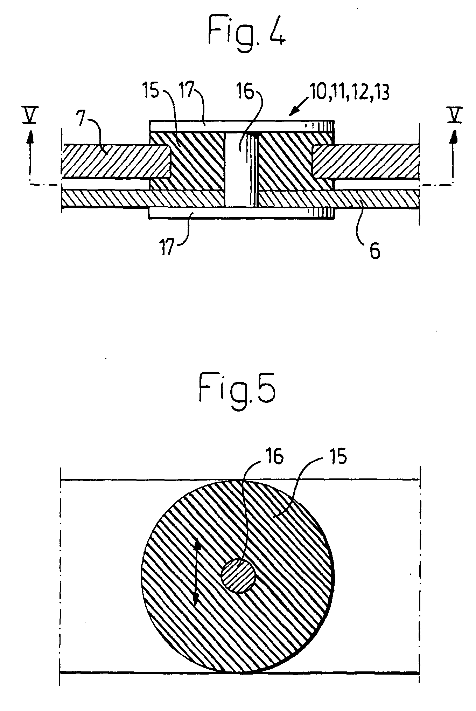

In der FIG 4 ist das federnde Verbindungsmittel 10 bis 13 gezeigt. Das federnde Verbindungsmittel 10 bis 13 umfaßt eine Gummibuchse 15, die mit der Halterung 7 fest verbunden ist, und eine Welle 16, die mit der Kompressionsplatte 6 fest verbunden ist, und die Kompressionsplatte 6 mit der Gummibuchse 15 und damit mit der Halterung 7 verbindet. Die beiden Enden der Welle 16 sind mit Abdeckscheiben 17 versehen.

In der FIG 5, die ein Schnitt entlang der Schnittlinie V-V in der FIG 4 zeigt, ist gezeigt, daß die Welle 16 bei einer Kompression in der Gummibuchse 15 so verschoben werden kann, daß die Gummibuchse 15 deformiert wird. Nach einer abgeschlossenen Kompression kehrt die Gummibuchse 15 in ihre ursprüngliche Form zurück.

Die Gummibuchse 15 kann im Rahmen der Erfindung auch mit der Kompressionsplatte 6 und die Welle 16 mit der Halterung verbunden sein.

In der FIG 7 ist in einem weiteren Ausführungsbeispiel gezeigt, daß die federnden Verbindungsmittel 10 und 12 an der Querseite der Halterung 7 angebracht sein können.

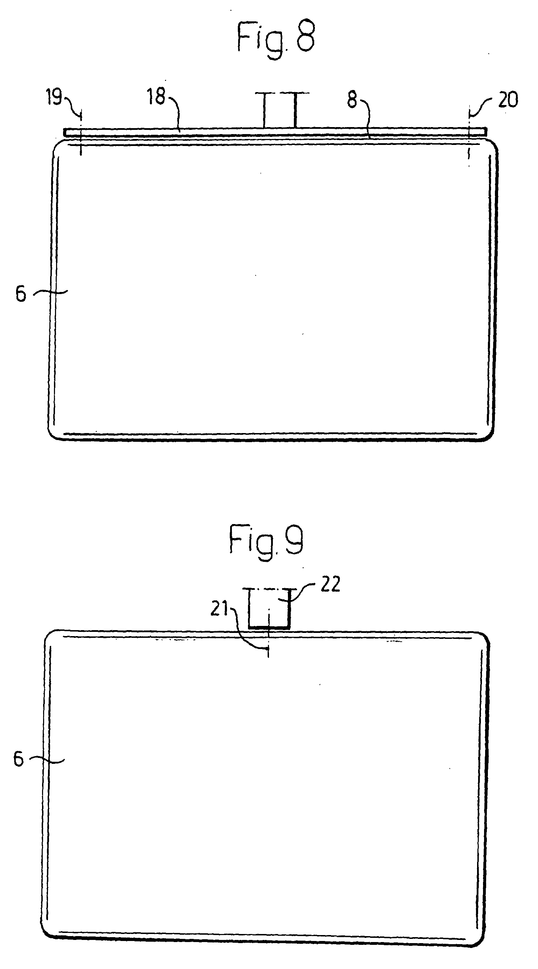

In der FIG 8 ist eine Halterung 18 gezeigt, die sich lediglich entlang der Querseite 8 der Kompressionsplatte 6 erstreckt, wobei federnde Verbindungsmittel 19, 20 mit der Kompressionsplatte 6 im Bereich der Endseiten der Halterung 18 verbunden sind.

In der FIG 9 ist eine besonders einfache Ausführungsform der Erfindung gezeigt, in der ein einziges federndes Verbindungsmittel 21 in der Mitte derjenigen Seite der Kompressionsplatte 6, die gegen die Halterung 22 gerichtet ist, d.h. der Querseite 8, angeordnet ist.

Auch durch die in den FIG 7 - 9 gezeigten Ausführungsbeispiele der Erfindung kann wie beschrieben mit Hilfe einer Halterung und einer Kompressionsplatte auf eine einfache und billige Weise eine gleichmäßige Verteilung der Kraft gegen die Brust, die untersucht werden soll, erreicht werden.

## Patentansprüche

1. Röntgendiagnostikgerät für Mammographieuntersuchungen mit einem Arm (2) für eine Röntgenröhre (3) und einem Objekttisch (4) und einer zwischen der Röntgenröhre (3) und dem Objekttisch (4) angeordneten Kompressionsplatte (6), die über eine Halterung (7, 18, 22) mit dem Arm (2) verbunden und entlang diesem verschiebbar ist, wobei die Kompressionsplatte (6) gegenüber dem Objekttisch (4) in Längs- und Querrichtung der Kompressionsplatte (6) drehbar ist, **dadurch gekennzeichnet, daß** die Kompressionsplatte (6) mittels mindestens eines federnden Verbindungsmittels (10-13, 19-21) in mindestens einem Verbindungspunkt derart mit der Halterung (7, 18, 22) verbunden ist, dass die Kompressionsplatte (6) gegenüber der Halterung (7, 18, 22) in Längs- und Querrichtung drehbar ist.

2. Röntgendiagnostikgerät nach Anspruch 1, **dadurch gekennzeichnet, daß** ein einziges Verbindungsmittel (21) in der Mitte derjenigen Seite der Kompressionsplatte (6), die gegen die Halterung (22) gerichtet ist, angeordnet ist.

3. Röntgendiagnostikgerät nach Anspruch 1, **dadurch gekennzeichnet, daß** zwei Verbindungsmittel (19, 20) auf jeweils einer Seite der Mitte der gegen die Halterung (18) gerichteten Seite (8) der Kompressionsplatte (6) angeordnet sind.

4. Röntgendiagnostikgerät nach Anspruch 2 oder 3, **dadurch gekennzeichnet, daß** die Halterung (7) U-förmig ausgebildet ist und entlang der einen Querseite (8) der Kompressionsplatte (6) sowie entlang deren beiden Längsseiten (9) verläuft, wobei jede Längsseite (9) der Kompressionsplatte (6) mit der Halterung (7) mittels mindestens eines Verbindungsmittels (11, 13) verbunden ist.

5. Röntgendiagnostikgerät nach Anspruch 1, **dadurch gekennzeichnet, daß** die Halterung (7) U-förmig ausgebildet ist und entlang der einen Querseite (8) der Kompressionsplatte (6) sowie entlang deren beiden Längsseiten (9) verläuft, wobei jede Längsseite (9) der Kompressionsplatte (6) mit der Halterung (7) mittels zweier Verbindungsmittel (10-13) verbunden ist.

6. Röntgendiagnostikgerät nach einem der Ansprüche 1 bis 5, **dadurch gekennzeichnet, daß** die Halterung (7, 18, 22) mit mindestens einer Gummibuchse (15) versehen ist und daß die Kompressionsplatte (6) mit der Gummibuchse (15) über eine Welle (16), die an der Kompressionsplatte (6) befestigt ist, verbunden ist.

7. Röntgendiagnostikgerät nach einem der Ansprüche 1 bis 5, **dadurch gekennzeichnet, daß** die Kompressionsplatte (6) mit mindestens einer Gummibuchse (15) versehen ist und daß die Halterung (7, 18, 22) mit der Gummibuchse (15) über eine Welle (16), die an der Halterung (7, 18, 22) befestigt ist, verbunden ist.

## Claims

1. An X-ray mammography apparatus with an arm (2) for an X-ray tube (3) and a subject table (4) and a compression plate (6) arranged between the X-ray tube (3) and the subject table (4), connected to the arm (2) via a mount (7, 18, 22) and able to be moved along said arm, with the compression plate (6) being able to be rotated in a longitudinal direction and a transverse direction in relation to the subject table (4), **characterized in that** the compression plate (6) is connected by at least one resilient connecting element (10-13, 19-21) at at least one connection point to the mount (7, 18, 22) such that the compression plate (6) can be rotated in a longitudinal direction and in a transverse direction in relation to the mount (7, 18, 22).

2. An X-ray mammography apparatus in accordance with Claim 1, **characterized in that** a single connecting element (21) is arranged in the center of that side of the compression plate directed towards the mount (22).

3. An X-ray mammography apparatus in accordance with Claim 1, **characterized in that** at least one connecting elements (19, 20) are arranged at opposite sides of a center of the side (8) of that compression plate (6) directed towards the mount (18).

4. An X-ray mammography apparatus in accordance with Claim 2 or 3, **characterized in that** the mount (7) is U-shaped and proceeds along one transverse side (8) of the compression plate (6) and two longitudinal sides (9) of the compression plate, with each longitudinal side (9) of the compression plate (6) being connected to the mount (7) by at least one connecting element (11, 13).

5. An X-ray mammography apparatus in accordance with Claim 1, **characterized in that** the mount (7) is U-shaped and proceeds along one transverse side (8) of the compression plate (6) and along both longitudinal sides (9) of the compression plate, with each longitudinal side (9) of the compression plate (6) being connected to the mount (7) by two connecting elements (10-13).

6. An X-ray mammography apparatus in accordance with one of the Claims 1 to 5, **characterized in that** the mount (7, 18, 22) is provided with at least one rubber bushing (15) and that the compression plate (6) is connected to the rubber bushing (15) by a shaft (16) which is attached to the compression plate (6).

7. An X-ray mammography apparatus in accordance with one of the Claims 1 to 5, **characterized in that** the compression plate (6) is provided with at least one rubber bushing (15) and that the mount (7, 18, 22) is connected to the rubber bushing (15) by a shaft (16) which is attached to the mount (7, 18, 22).

## Revendications

1. Appareil de diagnostic à rayons X pour des examens de mammographie, comprenant un bras (2) pour un tube (3) radiogène et une tablette (4) objet et une plaque (6) de compression, qui est disposée entre le tube (3) radiogène et la tablette (4) objet qui est reliée par une fixation (7, 18, 22) au bras (2) et qui peut coulisser le long de celui-ci, la plaque (6) de compression pouvant tourner par rapport à la tablette (4) objet dans la direction longitudinale et transversale de la plaque (6) de compression, **caractérisé en ce que** la plaque (6) de compression est reliée au moyen d'au moins un moyen ( 10 à 13 ; 19 à 21) élastique de liaison en au moins un point de liaison à la fixation (7, 18, 22) de sorte que la plaque (6) de compression peut tourner dans la direction longitudinale et dans la direction transversale par rapport à la fixation (7, 18, 22).

2. Appareil de diagnostic à rayons X, suivant la revendication 1, **caractérisé en ce qu'**un moyen (21) unique de liaison est disposé au milieu du côté de la plaque (6) de compression, qui est tourné vers la fixation (22).

3. Appareil de diagnostic à rayons X, suivant la revendication 1, **caractérisé en ce que** deux moyens (19, 20) de liaison sont disposés respectivement d'un côté du milieu du côté (8) de la plaque (6) de la plaque de compression, qui est tourné vers la fixation (18).

4. Appareil de diagnostic à rayons X, suivant la revendication 2 ou 3, **caractérisé en ce que** la fixation (7) est en forme de U et s'étend le long d'un côté (8) transversal de la plaque (6) de compression, ainsi que le long de ses deux côtés (9) longitudinaux, chaque côté (9) longitudinal de la plaque (6) de compression étant relié à la fixation (7) au moyen d'au moins un moyen (11, 13) de liaison.

5. Appareil de diagnostic à rayons X, suivant la revendication 1,.**caractérisé en ce que** la fixation (7) est en forme de U et s'étend le long de l'un des côtés (8) transversaux de la plaque (6) de compression, ainsi que le long de ses deux côtés (9) longitudinaux, chaque côté (9) longitudinal de la plaque (6) de compression étant relié à la fixation (7) à l'aide d'au moins deux moyens (10 à 13) de liaison.

6. Appareil de diagnostic à rayons X, suivant l'une des revendications 1 à 5, **caractérisé en ce que** la fixation (7, 18, 22) est munie d'au moins une douille (15) en caoutchouc et **en ce que** la plaque (6) de compression est reliée à la douille (15) de caoutchouc par un arbre (16) qui est fixé à la plaque (6) de compression.

7. Appareil de diagnostic à rayons X, suivant l'une des revendications 1 à 5, **caractérisé en ce que** la plaque (6) de compression est munie d'au moins une douille (15) en caoutchouc et **en ce que** la fixation (7, 18, 22) est reliée à la douille (15) de caoutchouc par un arbre (15) qui est fixé à la fixation (7, 18, 22).
